# EUROPEAN PATENT SPECIFICATION

(11) **EP 4 014 968 B1**
(45) Date of publication and mention of the grant of the patent: **29.01.2025**
(21) Application number: 21214648.4
(22) Date of filing: 15.12.2021
(51) Int. Cl.: A61K 9/16, A61K 9/20, A61K 9/28, A61K 31/505

(54) **A FILM COATED TABLET COMPRISING MICRONIZED AMBRISENTAN**
FILMBESCHICHTETE TABLETTE MIT MIKRONISIERTEM AMBROSINTAN
COMPRIMÉ REVÊTU D'UN FILM COMPRENANT DE L'AMBRISENTAN MICRONISÉ

(30) Priority: 16.12.2020 TR 202020618
(43) Date of publication of application: 22.06.2022
(73) Proprietor: Sanovel Ilac Sanayi ve Ticaret A.S., Sisli - Istanbul (TR)
(72) Inventor: PEHLIVAN AKALIN, Nur, 34460 Istanbul (TR); GÜLER, Tolga, 34460 Istanbul (TR)
(74) Representative: Genç Ilhan, Oznur

(56) References cited:
- CN-A- 110 876 731
- US-A1- 2011 189 243
- US-A1- 2012 022 087
- EUROPÄISCHE ARZNEIMITTEL-AGENTUR (EMEA): "Volibris-H-C-839-II-06", INTERNET CITATION, 1 June 2008 (2008-06-01), pages 1 - 53, XP002553850, Retrieved from the Internet <URL:http://www.emea.europa.eu/humandocs/PDFs/EPAR/volibris/H-839-PI-de.pdf> [retrieved on 20090101]

## Description

### Field of the Invention

The present invention relates to a film coated tablet formulation comprising ambrisentan or pharmaceutically acceptable salts thereof wherein ambrisentan or pharmaceutically acceptable salts thereof has a d (0.9) particle size less than 10 µm and wherein said film coated tablet further comprises lactose monohydrate in an amount between 40.0% and 60.0% by weight of the total tablet formulation. Further, the present invention also relates to a simple, rapid, cost effective, time-saving and industrially convenient process.

### Background of the Invention

Pulmonary arterial hypertension (PAH) is a specific type of PH and is a serious, progressive, and life-threatening disease of the pulmonary vascular system characterized by extreme vasoconstriction and abnormal proliferation of smooth muscle cells in the pulmonary artery wall.

Pulmonary hypertension drugs are mainly divided into phosphodiesterase-5 (PDE-5) inhibitors, prostacyclin and endothelin receptor antagonists according to their different mechanisms of action. Among them, endothelin receptor antagonists can completely bind to endothelin receptors, thereby achieving the purpose of delaying the deterioration of the disease.

Ambrisentan, a potent selective endothelin receptor antagonist, is utilized for the treatment of pulmonary arterial hypertension and idiopathic pulmonary fibrosis. It blocks the endothelin receptor on vascular smooth muscle cells and cardiac myocytes, preventing vasoconstriction and smooth muscle proliferation. Ambrisentan is chemically known as (S)-2-((4,6-dimethylpyrimidin-2-yl)oxy)-3-methoxy-3,3-diphenyl propanoic acid and has the structural formula as shown in Formula I.

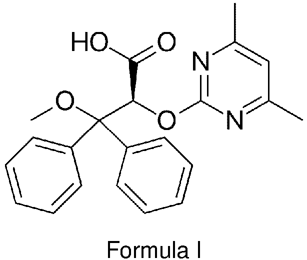

Ambrisentan is marketed under the trade name Letairis^{™} or Volibris^{®}.

Ambrisentan is a poorly soluble drug that is almost insoluble in water and 0.1mol·L-1 hydrochloric acid solution. In the actual production of tablets, it often encounters problems of low dissolution or even unqualified. Also, ambrisentan is used small proportion that can lead to considerable problems during the manufacture of the composition with regard to the uniformity of the content of active agent in the individual composition units. Because of problems uniformity of the content, the active substance may interact with several excipients. It reflects that content uniformity play important role in the dissolution of the drug.

There still remains a need in the prior art to provide an improved pharmaceutical tablet formulation comprising ambrisentan or pharmaceutically acceptable salts thereof.

At this application, a film coated tablet comprises ambrisentan or pharmaceutically acceptable salts thereof wherein ambrisentan or pharmaceutically acceptable salts thereof has a d (0.9) particle size less than 10 µm. Also, according to the invention said film coated tablet further comprises lactose monohydrate in an amount between 40.0% and 60.0% by weight of the total tablet formulation. Due to the use this film coated tablet of ambrisentan, problems seen in the prior art are overcome. So, this provides the desired solubility, content uniformity, compressibility of the tablet. Also, the film coated tablet has been developed by using standard techniques which is simple and cost-effective method.

### Detailed Description of the Invention

The main object of the present invention is to eliminate problems caused by ambrisentan and bringing additional advantages to the relevant prior art.

Another object of the present invention is to provide a film coated tablet comprising ambrisentan or pharmaceutically acceptable salts thereof having the desired dissolution profile, stability and content uniformity.

Another object of the present invention is to provide a film coated tablet having the desired compressibility.

We have found that ambrisentan or pharmaceutically acceptable salts thereof having the following particle sizes is very important for formulation. Especially, it positively affects the dissolution properties and powder homogenization. The obtained tablets have the desired dissolution profile. The powder is more homogeneous. The content uniformity of the tablets obtained from the more homogeneous powder is more ideal. Therefore, it provides better bioavailability.

As used here in, 'particle size' means the cumulative volume size distrubition as tested by any conventionally accepted method such as the laser diffraction method (i.e. malvern analysis). The term d (0.1) means, the size at which 10% by volume of the particles are finer and d (0.5) means the size at which 50% by volume of the particles are finer and d (0.9) means the size at which %90 by volume of the particles are finer.According to the present invention, the film coated tablet comprises ambrisentan or pharmaceutically acceptable salts thereof wherein ambrisentan or pharmaceutically acceptable salts thereof has a d (0.9) particle size less than 10 µm; wherein the particle size (d(0.9)) is determined by laser diffraction method (Malvern analysis) and the term d (0.9) refers to particle size at a volume cumulative 90% and at which %90 by volume of the particles are finer than 10 µm

According to another embodiment of the present invention, ambrisentan is present in the form of free base.

According to another embodiment of the present invention, ambrisentan or pharmaceutically acceptable salts thereof has a d (0.9) particle size less than 8 µm, preferably less than 6 µm.

According to another embodiment of the present invention, ambrisentan or pharmaceutically acceptable salts thereof has a d (0.5) particle size less than 6 µm, preferably less than 3 µm.

According to another embodiment of the present invention, ambrisentan or pharmaceutically acceptable salts thereof has a d (0.1) particle size less than 3 µm, preferably less than 1 µm.

According to another embodiment of the present invention, ambrisentan or pharmaceutically acceptable salts thereof has a d (0.9) particle size less than 8 µm, a d (0.5) particle size less than 6 µm, a d (0.1) particle size less than 3 µm.

According to another embodiment of the present invention, the amount of ambrisentan or pharmaceutically acceptable salts is between 1.0% and 8.0% by weight in the total film coated tablet.

According to another embodiment of the present invention, the amount of ambrisentan or pharmaceutically acceptable salts is between 2.0% and 7.0% by weight in the total film coated tablet.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agents and the excipients.

According to another embodiment of the present invention, the film coated tablet further comprises pharmaceutically acceptable excipients are selected from the group comprising fillers, disintegrants, lubricants/glidants, film coating agents or mixtures thereof.

According to the invention; the filler is lactose monohydrate and lactose monohydrate is in an amount between 40.0% and 60.0% by weight of the total tablet formulation.

According to another embodiment, additional fillers are selected from group comprising microcrystalline cellulose, ammonium alginate, calcium carbonate, calcium phosphate, calcium phosphate dehydrate, neutral pellets, calcium sulfate, cellulose, cellulose acetate, dextrose, erythritol, ethylcellulose, fructose, glyceryl palmitostearate, lactose, mannitol, magnesium carbonate, magnesium oxide, maltodextrin, maltose, polydextrose, polymethacrylates, polyvinylpyrrolidone, simethicone, sodium alginate, sodium chloride, sorbitol, starch, sucrose, sugar spheres, sulfobutylether beta-cyclodextrin or mixtures thereof.

According to another embodiment of the present invention, microcrystalline cellulose is used in combination with lactose monohydrate.

According to another embodiment of the present invention, the amount of microcrystalline cellulose is between 15.0% and 30.0% by weight of the total tablet formulation.

Suitable disintegrants are selected from the group comprising croscarmellose sodium, crospovidone, alginates, magnesium aluminium silica, sodium carboxymethyl cellulose, calcium silicate, sodium starch glycolate, carboxymethyl cellulose, calcium carboxymethyl cellulose, low substituted hydroxypropyl cellulose, polyacryline potassium, poloxamer, sodium alginate, sodium glycine carbonate, sodium dodecyl sulfate or mixtures thereof.

According to another embodiment of the present invention, the disintegrant is croscarmellose sodium.

According to another embodiment of the present invention, the amount of disintegrants is between 1.0% and 5.0% by weight of the total tablet formulation.

Suitable lubricants/glidants are selected from the group comprising magnesium stearate, sodium stearyl fumarate, colloidal silicon dioxide, calcium stearate, talc, sodium chlorate, magnesium lauryl sulfate, sodium oleate, sodium acetate, sodium benzoate, polyethylene glycol, stearic acid, fatty acid, fumaric acid, glyceryl palmito sulphate, sodium lauryl sulphate, aluminium silicate, colloidal silica, calcium silicate, magnesium silicate, magnesium oxide, starch or mixtures thereof.

According to another embodiment of the present invention, the lubricants/glidants is magnesium stearate.

According to another embodiment of the present invention, the amount of lubricants/glidants is between 0.1% and 2.0% by weight of the total tablet formulation.

In the present invention, the tablet comprises film coating to protect the composition against the moisture and light to maintain the stability. Suitable film coating agents are selected from the group comprising hydroxypropylmethylcellulose, polyethylene glycol (PEG), talc, titanium dioxide, polyvinyl alcohol (PVA), polyvinyl alcohol-polyethylene glycol copolymers, polyvinylprolidone, lecithin, polyvinylprolidone-vinyl acetate copolymer (PVP-VA), pigments, dyes, iron oxide or mixtures thereof.

According to another embodiment of the present invention, film coating agents are polyethylene glycol (PEG-3350, PEG-8000), talc, titanium dioxide, polyvinyl alcohol (PVA), lecithin. These agents provide the desired mechanical stability of the tablet.

It has surprisingly been found that the film coated tablet of excellent uniformity and showing improved compressibility can be obtained when ambrisentan or pharmaceutically acceptable salts thereof and all excipients (except for lubricants/glidants) are formulated together in wet granulation process. Wet granulation process efficiently counteracts segregation, so it can achieve good dissolution and disintegration properties. Solvents are used in the process.

Suitable solvents are selected from a group comprising pure water, ethyl alcohol, glycerin, sorbitol, polyethylene glycol, propylene glycol, isopropyl alcohol, or mixtures thereof, preferably granulation solution is pure water or ethyl alcohol or mixtures thereof. Preferably, wet granulation solution is pure water and ethyl alcohol (%96).

According to another embodiment of the present invention, the film coated tablet comprises;
1.0-8.0% by weight of ambrisentan or pharmaceutically acceptable salts thereof
40.0-60.0% by weight of lactose monohydrate
15.0-30.0% by weight of microcrystalline cellulose
1.0% - 5.0% by weight of croscarmellose sodium
0.1% - 2.0% by weight of magnesium stearate
1.0% - 6.0% by weight of film coating agent in the total tablet formulation; wherein d (0.9) particle size of ambrisentan or pharmaceutically acceptable salts thereof is less than 10 µm.

According to another embodiment of the present invention, a process for the preparing the film coated tablet processed wet granulation comprise the following steps:
a) Sieving ambrisentan, lactose monohydrate, microcrystalline cellulose and croscarmellose sodium and mixing ay wet granulator,
b) Adding ethyl alcohol (%96):pure water (60:40) mixture and then granulating,
c) Sieving the mixture through 8 mm and then drying the mixture at fluidized bed,
d) Sieving the mixture through 1.5 mm,
e) Adding magnesium stearate and then mixing,
f) Compressing the mixture to form tablets,
g) Coating the tablets with film coating.

### Example 1: A film coated tablet comprising ambrisentan or pharmaceutically acceptable salts thereof

| **Ingredients** | **Amount (% by weight of the total)** |
|---|---|
| Ambrisentan | 1.0 - 8.0 |
| Lactose monohydrate | 40.0 - 60.0 |
| Microcrystalline cellulose | 15.0 - 30.0 |
| Croscarmellose sodium | 1.0 - 5.0 |
| Magnesium stearate | 0.1 - 2.0 |
| Ethyl alcohol | 5.0 - 20.0 |
| Pure water | 5.0 - 15.0 |
| Film coating agents | 1.0 - 6.0 |
| **Total composition** | **100** |

### Example 2: A film coated tablet comprising ambrisentan

| **Ingredients** | **Amount (% by weight of the total)** |
|---|---|
| Ambrisentan | 2.6 |
| Lactose monohydrate | 50.2 |
| Microcrystalline cellulose | 21.1 |
| Croscarmellose sodium | 2.1 |
| Magnesium stearate | 0.53 |
| Ethyl alcohol | 11.11 |
| Pure water | 9.5 |
| Film coating agents | 2.6 |
| **Total composition** | **100** |

### Example 3: A film coated tablet comprising ambrisentan

| Ingredients | **Amount** (% **by weight of the total)** |
|---|---|
| Ambrisentan | 5.2 |
| Lactose monohydrate | 47.6 |
| Microcrystalline cellulose | 21.1 |
| Croscarmellose sodium | 2.1 |
| Magnesium stearate | 0.53 |
| Ethyl alcohol | 11.11 |
| Pure water | 9.5 |
| Film coating agents | 2.6 |
| **Total composition** | **100** |

### Process for example 1, 2, 3;

a) Sieving ambrisentan, lactose monohydrate, microcrystalline cellulose and croscarmellose sodium and mixing ay wet granulator,
b) Adding ethyl alcohol (%96):pure water (60:40) mixture and then granulating,
c) Sieving the mixture through 8 mm and then drying the mixture at fluidized bed,
d) Sieving the mixture through 1.5 mm,
e) Adding magnesium stearate and then mixing,
f) Compressing the mixture to form tablets,
g) Coating the tablets with film coating.

### Film coating agents

| | |
|---|---|
| Polyvinyl alcohol | %45.2 |
| Titanium dioxide | %29.85 |
| Polyethylene glycol 3350 | %10.7 |
| Talc | %6.8 |
| Polyethylene glycol 8000 | %5.3 |
| Lecithin | %2.0 |
| FD&C #40/Allura Red | %1.5 |
| **Aluminum** Lake | |

## Claims

1. A film coated tablet comprising ambrisentan or pharmaceutically acceptable salts thereof wherein ambrisentan or pharmaceutically acceptable salts thereof has a d (0.9) particle size less than 10 µm, wherein the particle size (d(0.9)) is determined by laser diffraction method (Malvern analysis) and the term d (0.9) refers to particle size at a volume cumulative 90% and at which 90% by volume of the particles are finer than 10 µm;
wherein said film coated tablet further comprises lactose monohydrate in an amount between 40.0% and 60.0% by weight of the total tablet formulation.

2. The film coated tablet according to claim 1, wherein ambrisentan or pharmaceutically acceptable salts thereof has a d (0.9) particle size less than 8 µm, preferably less than 6 µm.

3. The film coated tablet according to claim 1, wherein ambrisentan or pharmaceutically acceptable salts thereof has a d (0.5) particle size less than 6 µm, preferably less than 3 µm.

4. The film coated tablet according to claim 1, wherein ambrisentan or pharmaceutically acceptable salts thereof has a d (0.1) particle size less than 3 µm, preferably less than 1 µm.

5. The film coated tablet according to claim 1, wherein ambrisentan or pharmaceutically acceptable salts thereof has a d (0.9) particle size less than 8 µm, a d (0.5) particle size less than 6 µm, a d (0.1) particle size less than 3 µm.

6. The film coated tablet according to claim 1, wherein the amount of ambrisentan or pharmaceutically acceptable salts is between 2.0% and 7.0% by weight in the total film coated tablet.

7. The film coated tablet according to claim 1, further comprising pharmaceutically acceptable excipients are selected from the group comprising fillers, disintegrants, lubricants/glidants, film coating agents or mixtures thereof.

8. The film coated tablet according to claim 7, wherein disintegrants are selected from the group comprising croscarmellose sodium, crospovidone, alginates, magnesium aluminium silica, sodium carboxymethyl cellulose, calcium silicate, sodium starch glycolate, carboxymethyl cellulose, calcium carboxymethyl cellulose, low substituted hydroxypropyl cellulose, polyacryline potassium, poloxamer, sodium alginate, sodium glycine carbonate, sodium dodecyl sulfate or mixtures thereof.

9. The film coated tablet according to claim 8, wherein the amount of disintegrants is between 1.0% and 5.0% by weight of the total tablet formulation.

10. The film coated tablet according to claim 7, wherein lubricants/glidants are selected from the group comprising magnesium stearate, sodium stearyl fumarate, colloidal silicon dioxide, calcium stearate, talc, sodium chlorate, magnesium lauryl sulfate, sodium oleate, sodium acetate, sodium benzoate, polyethylene glycol, stearic acid, fatty acid, fumaric acid, glyceryl palmito sulphate, sodium lauryl sulphate, aluminium silicate, colloidal silica, calcium silicate, magnesium silicate, magnesium oxide, starch or mixtures thereof.

11. The film coated tablet according to claim 7, comprising;
1.0-8.0% by weight of ambrisentan or pharmaceutically acceptable salts thereof
40.0-60.0% by weight of lactose monohydrate
15.0-30.0% by weight of microcrystalline cellulose
1.0% - 5.0% by weight of croscarmellose sodium
0.1% - 2.0% by weight of magnesium stearate
1.0% - 6.0% by weight of film coating agent in the total tablet formulation.

12. A process for preparing the film coated tablet according to any one of claims 1 to 11 by wet granulation comprising the following steps:
a) Sieving ambrisentan, lactose monohydrate, microcrystalline cellulose and croscarmellose sodium and mixing ay wet granulator,
b) Adding ethyl alcohol (96%) :pure water (60:40) mixture and then granulating,
c) Sieving the mixture through 8 mm and then drying the mixture at fluidized bed,
d) Sieving the mixture through 1.5 mm,
e) Adding magnesium stearate and then mixing,
f) Compressing the mixture to form tablets,
g) Coating the tablets with film coating.

## Patentansprüche

1. Filmbeschichtete Tablette, umfassend Ambrisentan oder pharmazeutisch verträgliche Salze davon, wobei Ambrisentan oder pharmazeutisch verträgliche Salze davon eine d(0,9)- Partikelgröße von weniger als 10 µm aufweisen, wobei die Partikelgröße (d(0,9)) durch ein Laserbeugungsverfahren (Malvern-Analyse) bestimmt wird und sich der Begriff d (0,9) auf die Partikelgröße bei einem Volumen von kumulativen 90% bezieht, bei dem 90 Vol.-% der Partikeln feiner als 10 µm sind;
wobei die filmbeschichtete Tablette weiterhin Lactosemonohydrat in einer Menge zwischen 40,0 und 60,0 Gew.-% der gesamten Tablettenformulierung enthält.

2. Filmbeschichtete Tablette nach Anspruch 1, wobei Ambrisentan oder pharmazeutisch verträgliche Salze davon eine d (0,9)-Partikelgröße von weniger als 8 µm, vorzugsweise weniger als 6 µm, aufweisen.

3. Filmbeschichtete Tablette nach Anspruch 1, wobei Ambrisentan oder pharmazeutisch verträgliche Salze davon eine d (0,5)-Partikelgröße von weniger als 6 µm, vorzugsweise weniger als 3 µm, aufweisen.

4. Filmbeschichtete Tablette nach Anspruch 1, wobei Ambrisentan oder pharmazeutisch verträgliche Salze davon eine d (0,1)-Partikelgröße von weniger als 3 µm, vorzugsweise weniger als 1 µm, aufweisen.

5. Filmbeschichtete Tablette nach Anspruch 1, wobei Ambrisentan oder pharmazeutisch verträgliche Salze davon eine d (0,9) Partikelgröße von weniger als 8 µm, eine d (0,5) Partikelgröße von weniger als 6 µm, eine d (0,1) Partikelgröße von weniger als 3 µm aufweisen.

6. Filmbeschichtete Tablette nach Anspruch 1, wobei die Menge an Ambrisentan oder pharmazeutisch akzeptablen Salzen zwischen 2,0 und 7,0 Gew.-% der gesamten filmbeschichteten Tablette beträgt.

7. Filmbeschichtete Tablette nach Anspruch 1 enthält ferner pharmazeutisch verträgliche Hilfsstoffe, die aus der Gruppe ausgewählt sind, die Füllstoffe, Sprengmittel, Schmiermittel/Gleitmittel, Filmüberzugsmittel oder Mischungen davon umfasst.

8. Filmbeschichtete Tablette nach Anspruch 7, wobei die Sprengmittel aus der Gruppe ausgewählt sind, die Croscarmellose-Natrium, Crospovidon, Alginate, Magnesiumaluminiumsiliciumdioxid, Natriumcarboxymethylcellulose, Calciumsilicat, Natriumstärkeglykolat, Carboxymethylcellulose, Calciumcarboxymethylcellulose, niedrig substituierte Hydroxypropylcellulose, Polyacrylinkalium, Poloxamer, Natriumalginat, Natriumglycincarbonat, Natriumdodecylsulfat oder Mischungen davon.

9. Filmbeschichtete Tablette nach Anspruch 8, wobei die Menge der Sprengmittel zwischen 1,0 und 5,0 Gew.-% der gesamten Tablettenformulierung beträgt.

10. Filmbeschichtete Tablette nach Anspruch 7, wobei die Gleitmittel aus der Gruppe Magnesiumstearat, Natriumstearylfumarat, kolloidales Siliciumdioxid, Calciumstearat, Talk, Natriumchlorat, Magnesiumlaurylsulfat, Natriumoleat, Natriumacetat, Natriumbenzoat, Polyethylenglykol, Stearinsäure, Fettsäure, Fumarsäure, Glycerylpalmitosulfat, Natriumlaurylsulfat, Aluminiumsilicat, kolloidales Siliciumdioxid, Calciumsilicat, Magnesiumsilicat, Magnesiumoxid, Stärke oder Mischungen davon.

11. Filmbeschichtete Tablette nach Anspruch 7, umfassend;
1,0-8,0 Gew.-% Ambrisentan oder pharmazeutisch akzeptable Salze davon
40,0-60,0 GHT Laktosemonohydrat
15,0-30,0 Gew.-% mikrokristalline Cellulose
1,0 - 5,0 Gew.-% Croscarmellose-Natrium
0,1 - 2,0 Gew.-% Magnesiumstearat
1,0 - 6,0 Gew.-% Filmüberzugsmittel in der gesamten Tablettenformulierung.

12. Verfahren zur Herstellung der filmbeschichteten Tablette nach einem der Ansprüche 1 bis 11 durch Nassgranulation, umfassend die folgenden Schritte:
a) Sieben von Ambrisentan, Laktosemonohydrat, mikrokristalliner Cellulose und Croscarmellose-Natrium und Mischen in einem Nassgranulator,
b) Zugabe von Ethylalkohol (96%): reines Wasser (60:40) Gemisch und anschließendes Granulieren,
c) Sieben der Mischung durch 8 mm und anschließendes Trocknen der Mischung im Fließbett,
d) Siebung der Mischung durch 1,5 mm,
e) Hinzufügen von Magnesiumstearat und anschließendes Mischen,
f) Komprimieren der Mischung zu Tabletten,
g) Beschichtung der Tabletten mit einem Filmüberzug.

## Revendications

1. - Comprimé pelliculé comprenant de l'ambrisentan ou des sels pharmaceutiquement acceptables de celui-ci, dans lequel l'ambrisentan ou les sels pharmaceutiquement acceptables de celui-ci ont une taille de particule d (0,9) inférieure à 10 µm, dans lequel la taille de particule (d(0,9)) est déterminée par la méthode de diffraction laser (analyse Malvern) et le terme d (0,9) se réfère à une taille de particule à un volume cumulé de 90 % et auquel 90 % en volume des particules sont plus fines que 10 µm ;
dans lequel ledit comprimé pelliculé comprend en outre du lactose monohydraté dans une quantité entre 40,0 % et 60,0 % en poids de la formulation totale du comprimé.

2. - Comprimé pelliculé selon la revendication 1, dans lequel l'ambrisentan ou les sels pharmaceutiquement acceptables de celui-ci ont une taille de particule d (0,9) inférieure à 8 µm, de préférence inférieure à 6 µm.

3. - Comprimé pelliculé selon la revendication 1, dans lequel l'ambrisentan ou les sels pharmaceutiquement acceptables de celui-ci ont une taille de particule d (0,5) inférieure à 6 µm, de préférence inférieure à 3 µm.

4. - Comprimé pelliculé selon la revendication 1, dans lequel l'ambrisentan ou les sels pharmaceutiquement acceptables de celui-ci ont une taille de particule d (0,1) inférieure à 3 µm, de préférence inférieure à 1 µm.

5. - Comprimé pelliculé selon la revendication 1, dans lequel l'ambrisentan ou les sels pharmaceutiquement acceptables de celui-ci ont une taille de particule d (0,9) inférieure à 8 µm, une taille de particule d (0,5) inférieure à 6 µm, une taille de particule d (0,1) inférieure à 3 µm.

6. - Comprimé pelliculé selon la revendication 1, dans lequel la quantité d'ambrisentan ou de sels pharmaceutiquement acceptables est entre 2,0 % et 7,0 % en poids dans le comprimé pelliculé total.

7. - Comprimé pelliculé selon la revendication 1, comprenant en outre des excipients pharmaceutiquement acceptables choisis dans le groupe comprenant les charges, les agents de désintégration, les lubrifiants/agents de glissement, les agents de pelliculage ou les mélanges de ceux-ci.

8. - Comprimé pelliculé selon la revendication 7, dans lequel les agents de désintégration sont choisis dans le groupe comprenant la croscarmellose sodique, la crospovidone, les alginates, le silicate de magnésium et d'aluminium, la carboxyméthyl cellulose sodique, le silicate de calcium, le glycolate d'amidon sodique, la carboxyméthyl cellulose, la carboxyméthyl cellulose calcique, l'hydroxypropyl cellulose faiblement substituée, la polyacryline potassique, les poloxamères, l'alginate de sodium, le carbonate de glycine sodique, le dodécylsulfate de sodium ou les mélanges de ceux-ci.

9. - Comprimé pelliculé selon la revendication 8, dans lequel la quantité d'agents de désintégration est entre 1,0 % et 5,0 % en poids de la formulation totale du comprimé.

10. - Comprimé pelliculé selon la revendication 7, dans lequel les lubrifiants/agents de glissement sont choisis dans le groupe comprenant le stéarate de magnésium, le fumarate de stéaryle sodique, le dioxyde de silicium colloïdal, le stéarate de calcium, le talc, le chlorate de sodium, le lauryl sulfate de magnésium, l'oléate de sodium, l'acétate de sodium, le benzoate de sodium, le polyéthylène glycol, l'acide stéarique, les acides gras, l'acide fumarique, le palmito sulfate de glycéryle, le lauryl sulfate de sodium, le silicate d'aluminium, la silice colloïdale, le silicate de calcium, le silicate de magnésium, l'oxyde de magnésium, l'amidon ou les mélanges **de ceux-ci.**

11. - Comprimé pelliculé selon la revendication 7, comprenant :
de 1,0 à 8,0 % en poids d'ambrisentan ou de sels pharmaceutiquement acceptables de celui-ci
de 40,0 à 60,0 % en poids de lactose monohydraté
de 15,0 à 30,0% en poids de cellulose microcristalline
de 1,0 % à 5,0 % en poids de croscarmellose sodique
de 0,1 % à 2,0 % en poids de stéarate de magnésium
de 1,0 % à 6,0 % en poids d'agent de pelliculage dans la formulation totale du comprimé.

12. - Procédé de préparation du comprimé pelliculé selon l'une quelconque des revendications 1 à 11 par granulation par voie humide, comprenant les étapes suivantes :
a) Tamiser de l'ambrisentan, du lactose monohydraté, de la cellulose microcristalline et de la croscarmellose sodique et mélanger par granulateur par voie humide,
b) Ajouter un mélange alcool éthylique (96 %) : eau pure (60:40), puis granuler,
c) Tamiser le mélange à travers un tamis de 8 mm d'ouverture de maille puis sécher le mélange sur lit fluidisé,
d) Tamiser le mélange à travers un tamis de 1,5 mm d'ouverture de maille,
e) Ajouter du stéarate de magnésium, puis mélanger,
f) Comprimer le mélange pour former des comprimés,
g) Enrober les comprimés avec un pelliculage.
